# EUROPÄISCHE PATENTANMELDUNG

(11) **EP 1 655 031 A1**
(43) Veröffentlichungstag der Anmeldung: **10.05.2006**
(21) Anmeldenummer: 05022033.4
(22) Anmeldetag: 10.10.2005
(51) Int. Cl.: A61K 31/565, A61P 5/30, A61P 5/34

(54) **Dienogest enthaltendes Langzeit-Verfahren zur Empfängnisverhütung**

(30) Priorität: 08.10.2004 US 617293 P; 11.10.2004 EP 04024161
(71) Anmelder: Schering AG, 13342 Berlin (DE)
(72) Erfinder: Sachse, Andreas, 13465 Berlin (DE)
(74) Vertreter: Cramer, Eva-Maria

(57) **Zusammenfassung**

Ein Verfahren zur Verfahren für die weiblichen hormonale Empfängnisverhütung, wobei eine Einphasenzubereitung von Dienogest in einer täglichen Menge von 0,5 - 3,0 mg und Ethinylestradiol in einer täglichen Menge von 10 - 30 µg oder einem synthetischen oder natürlichen Estrogens, welches in estrogener Wirksamkeit äquivalent zu 10 - 30 µg Ethinylestradiol ist, über eine Zeitspanne von n x 21 Tagen, wobei n = 2 bis 5 ist, gefolgt von einer 7-tägigen Pillenpause,kontinuierlich verabreicht wird.

Es werden Blutungsprobleme vermieden, die mit der kontinuierlichen Anwendung hormonaler Empfängnisverhütungsmittel, andere Progestine als Dienoges enthaltend assoziiert sind. Gleichzeitig können mit dem erfindungsgemäßen Verfahren Akne, Dysmenorrhöe und /oder Endometriose behandelt werden.

## Beschreibung

Die Erfindung betrifft ein Verfahren für die weibliche hormonale Empfängnisverhütung, wobei eine Einphasenzubereitung von Dienogest in einer täglichen Menge von 0,5 - 3,0 mg und Ethinylestradiol in einer täglichen Menge von 10 - 30 µg oder einem synthetischen oder natürlichen Estrogens, welches in estrogener Wirksamkeit äquivalent zu 10 - 30 µg Ethinylestradiol ist, über eine Zeitspanne von n x 21 Tagen, wobei n = 2 bis 5 ist, gefolgt von einer 7-tägigen Pillenpause, kontinuierlich verabreicht wird. Es werden Blutungsprobleme vermieden, die mit der kontinuierlichen Anwendung hormonaler Empfängnisverhütungsmittel, andere Progestine als Dienogest enthaltend, assoziiert sind. Gleichzeitig können mit dem erfindungsgemäßen Verfahren Akne. Dysmenorrhöe und /oder Endometriose behandelt werden.

### Allgemeiner Stand der Technik

Die erste groß angelegte Studie eines oralen Festlangzeit-Kontrazeptivum-Behandlungsschemas (d.h. eines Zyklus von 90 Tagen = 84 Tage aktive Pillen gefolgt von 6 hormonfreien (Placebopillen)) wurde von Loudon und seinen Kollegen im Jahre 1977 veröffentlicht (British Medical Journal, 1977; 2:487 - 490). Bei dieser Studie wurde ein Einphasen-OC [50 µg Ethinylestradiol (EE)t2,5 mg Lynestrenol] angewendet. Trotz der hohen EE-Dosis wurden während der Langzeitzyklen bei einer bemerkenswerten Anzahl von Frauen Intermenstrualblutungen beobachtet, die mit jedem 90-Tagezyklus abnahmen. i 27 % der Patientinnen bekamen Intermenstrualschmierblutungen und Durchbruchblutungen während des ersten Dreimonatzyktus vor, die im vierten Langzeitzyklus auf 4 % (ausschließlich Schmierblutungen) abfielen. Intermenstrualblutungen waren 11 % der aus der Studie Ausscheidenden zuzuschreiben.

Siebzehn Jahre später wurde eine geplante Studie der kontinuierlichen Anwendung von 30 µg EE + 150 µg LNG (Nordette®) über 84 Tage, gefolgt von einer Woche Placebo von Kovacs et al (The British Journal of Family Planning, 1994; 19:274-275) veröffentlicht. Von den 203 Frauen. die an der Studie zu Beginn teilnahmen, durchliefen nur 59 (29,1 %) die ganze 12-monatige Behandlung (4 x 84 + 7 Tage). Der häufigste Grund des Abbruchs waren Durchbruchblutungen bei 73 Patientinnen (50,7 %) und Brustschmerzen sowie Kopfschmerzen bei jeweils 31 Patientinnen (21,5 %). Die Ausscheidungsrate lag während des ersten Langzeitzyklus (13 Wochen) am höchsten, während dem 34,5 % (n = 70) der registrierten Frauen absprangen, während die Ausscheidungsrate im zweiten bis vierten Langzeitzyklus (26, 39 + 52 Wochen) 21,8 % (43), 12,3 % (25) bzw. 3,0 % (6) betrug. Die Verfasser geben an, dass die Möglichkeit des Reduzierens der Häufigkeit der Menstruation von vielen Frauen zwar geschätzt wurde, dieser Vorteil jedoch bis zu einem gewissen Grad durch das häufige Vorkommen von Durchbruchblutungen zunichtegemacht wurde.

Hodgen offenbarte ein entsprechendes festgelegtes Behandlungsschema für die Anwendung von oralen Empfängnisverhütungsmitteln, das die Wirksamkeit aufrechterhalten sollte, während es eine verbesserte Kontrolle der Endometriumblutung bietet (US 5,898,032). Zusätzlich zu einer geringeren Menstrualblutung und Anämie der Patientinnen werden erhöhte Einhaltungsraten und eine bessere Lebensstilbequemlichkeit der Patientinnen als Vorteile dieser Methode aufgelistet. Den Ansprüchen gemäß wird eine Einphasenkombination eines Östrogens und Progestins kontinuierlich an 60 - 110 aufeinanderfolgenden Tagen verabreicht, gefolgt von 3 - 10 Tagen ohne Verabreichung. Die beanspruchten täglichen Mengen Östrogen und Progestin entsprechen 5 - 35 µg EE und 0,025 - 10 mg Norethindronacetat (NETA). Andere Progestine, wie Levonorgestrel (LNG), Desogestrel (DSG) oder 3-Ketodesogestrel sind ebenso beschrieben (jedoch befindet sich Dienogest nicht darunter). Das Gewichtsverhältnis der beiden aktiven Bestandteile (Ostrogen:Progestin) wird als mindestens 1:45 und bevorzugt mindestens 1:50 angegeben.

Ein entsprechendes 91 Tage-OC-Festlangzeitbehandlungsschema (84 Tage aktiv + 7 Tage Placebo) ist zum ersten Mal bei einem Multizentertest der Phase III von einem Jahr im Vergleich mit einem normalen 28 Tagezyklus (21 Tage aktiv + 7 Placebopillen) von Anderson et al. (Contraception, 2003; 68:89-96) geprüft worden. Es zeigte sich, dass das Langzeitzyklus-Behandlungsschema (jeweils 30 µg EE/150 µg LNG, Seasonale®, Barr Laboratories) zu einer größeren Anzahl von außerplanmäßigen (Durchbruch-) Intermenstrualblutungen von 37,6 Tagen im Vergleich mit 18,3 Tagen bei dem 28 Tage-Behandlungsschema führt. Die Gesamtzahl der planmäßigen (= Menstrualblutung) und außerplanmäßigen Blutungstage während des Jahres der Studie (364 Tage) 48,2 Tage beim Langzeitzyklus im Vergleich mit 50,8 Tagen beim normalen Zyklus betrug. Es wird berichtet, dass bei dem Langzeitzyklus die Durchbruchblutung (DBB) bei jedem nacheinanderfolgenden Zyklus (jeweils 84 + 7) von einem Mittelwert von 12 Tagen während Zyklus 1 auf einen Mittelwert von 4 Tagen während Zyklus 4 abfiel.

Die am häufigsten angegebenen Gründe für das Abbrechen der Studie waren Blutungen, Gewichtszunahme, Stimmungsumschläge und Akne ( = negative Vorfälle). Das Abbrechen auf Grund von unannehmbaren Blutungen war der Grund bei 7,7 % der dem 91-Tage-Behandlungsschema unterzogenen Patientinnen im Vergleich mit 1,8 % der Gruppe des 28-Tage-Behandlungsschemas. Die Gesamtausscheidungsraten betrugen jeweils 40,6 % bzw. 28,8 %.

Bei der klinischen Besprechung von NDA 21-544 (4. September, 2003) für Seasonafe® werden nicht nur die Ergebnisse für Seasonale® sondern auch die Ergebnisse für Seasonale® Ultra-Lo (20 µg EE/100 µg LNG pro Tag) berichtet. Es wurde festgestellt, dass das fixierte (84 + 7 Tage) Langzeitbehandlungsschema mit niedrigerer Dosis von EE zu einer noch schlechteren Blutungskontrolle führt.

Im Falle einer 12-monatigen randomisierten Multizenterstudie wurde die Akzeptanz der Langzeitanwendung (63 + 7 (pillenfreie) Tage) eines Einphasen-OCs mit 30 µm EE und 150 µg Desogestrel von Cachrimanidou im Vergleich mit dem entsprechenden Standardzyklusprodukt (21 + 7) untersucht (Contraception, 1993, 48:205 - 216). Es wurde festgestellt, das Durchbruchblutungen in der Langzeitzyklus- (n = 198) Gruppe signifikant häufiger auftraten im Vergleich mit der Standardzyklusgruppe (n=96). Entsprechend mehr Frauen, die das Langzeitzyklusprodukt einnahmen, schieden aus der Studie auf Grund von Blutungsproblemen (13 % im Vergleich mit 2 %, P < 0.01) aus. Bei beiden Gruppen nahmen die Intermenstrualblutungen (Durchbruch- und Schmierblutung) im Lauf der Zeit ab. Frauen, die bei Beginn der Studie schon OCs zu sich nahmen, wiesen im Vergleich mit Anfängerinnen wesentlich weniger häufige Episoden von Intermenstrualblutungen auf. Den Verfassern gemäß gaben eine relativ hohe Anzahl von Frauen ohne medizinische Gründe ihre Teilnahme an dem Versuch auf (17,2 % beim Langzeit-, 15,6 % beim Standardzyklus).

Hesch (US-Patentschrift 6,500,814) offenbart ein Niedrigdosis-Langzeitzyklusprodukt/-behandlungsschema, das dem Erfinder gemäß erstaunlicherweise eine hohe Empfängnisverhütungsverlässlichkeit sicherstellt und Intermenstrualblutungen verhindert. Zusätzlich dazu werden eine Reduzierung der mit OC verbundenen Nebenwirkungen (z.B. Thrombose) und eine vorteilhafte Auswirkung auf das prämenstruelle Syndrom (PMS) beschrieben. Des Weiteren sind die Prophylaxe und die Behandlung von Brustkrebs mit dem erfindungsgemäßen Produkt möglich. Hesch beansprucht die kontinuierliche und ununterbrochene Verabreichung eines kombinierten hormonalen Empfängnisverhütungsmittels für eine Zeitspanne von mehr als 110 Tagen. Es werden verschiedene natürliche oder synthetische Östrogene und Progestine (ausgenommen Dienogest) beschrieben. Wenn EE verwendet wird, so wird seine Dosis als zwischen 1 - 20 µg/Tag liegend beansprucht.

Kulmann (WO 02/22110) offenbart eine andere Methode für die hormonale Empfängnisverhütung, die die Anzahl von Entzugsblutungen reduziert, während sie eine verlässliche Empfängnisverhütung sicherstellt. Die Methode ist durch eine Reihenfolge aufeinanderfolgender Langzeitzyklen (= Einnahmeperiode) zunehmender Dauer gekennzeichnet. So kann die Patientin beispielsweise mit einer Einnahmeperiode von 21 aktiven Tabletten beginnen, gefolgt von 7 Placebos (21/7), auf die wiederum eine Einnahmeperiode von n x 42/7 folgt. Mit Ausnahme der abschließenden Einnahmeperiode ist die Dauer aller vorherigen Perioden vorherbestimmt (festgelegt). Erfindungsgemäß ist es auch möglich, die Hormondosis (verschiedene Progestine (einschließlich Dienogest) und/oder aufgelistete Östrogene) zwischen Einnahmeperioden sukzessive zu reduzieren.

Sulak et al. (Am J Obstet Gynecol, 2002;186:1142 - 1149) studierten rückblickend die Akzeptanz der Langzeitzyklusanwendung bei einer größeren Anzahl von Patientinnen mit Hormonentziehungssymptomen. Man erlaubte den Patientinnen, ihr 21 + 7-Standardbehandlungsschema durch Verlängern für eine spezifische Anzahl von Wochen, wie beispielsweise 6, 9 oder 12, oder Verlängern bis zum Durchbruchbluten oder bis zur Schmierblutungsentwicktung, 3 - 7-tägiges Unterbrechen und Wiederaufnahme zu ändern. In den Fällen, in denen sie 12 Wochen aktiver Pillenanwendung abschlossen hatten und ohne eine hormonfreie Pause weitermachen wollten, wurde ihnen das gestattet. Bei allen Patientinnen handelte es sich um vorherige Benutzer der Pille, die Einphasenpillen mit 30 - 35 µg Ethinylestradiol und eines der folgenden Progestine zu sich nahmen: Norethindron, Lavonorgestrel, Norgestimat oder Desogestrel. Von den 267 Patientinnen, die das Langzeitzyklus-Behandlungsschema begannen, entschlossen sich 57 (21 %), die Anwendung von OCs aus verschiedenen Gründen wie Verschlimmern von Nebenwirkungen einschließlich Übelkeit, Kopfschmerzen, Akne, Beinkrämpfen, hohem Blutdruck, Hefeinfektionen, Durchbruchblutungen und PMS (24 Patientinnen) und einem Wunsch nach Schwangerschaft (13 Patientinnen) abzubrechen. Von den 210 Patientinnen, die mit der Anwendung von OCs fortfuhren, entschlossen sich 38 (18 %) am häufigsten auf Grund von Durchbruchblutungen (11 Patientinnen), Druchbruch-Schmierblutungen (9) und schweren Entziehungsblutungen (2 Patientinnen), zum 21/7-Standardbehandlungsschema zurückzukehren.

Zahradnik und Moore (in: Elstein M, Extragenital effects of oral contraceptives (Extragenitalwirkung oraler Kontrazeptiva). Carnforth: Parthenon Publ. 1997; Seite 53 - 60) studierten die empfängnisverhütende Effizienz. Zyklusstabilität, Verträglichkeit und Einhaltung eines neuen Standardzyklus- (21 + 7 (tablettenfreie Zwischenzeit) Empfängnisverhütungsmittels, das 30 µg EE + 2 mg Dienogest (Valette®) enthielt. Es zeigte sich, dass die Schwere und Menge der Intermenstrualblutung, Schmierblutung und Durchbruchblutung im Laufe der einjährigen Studie der Phase III (12 Zyklen) abnahm. Außerdem wurde eine deutliche Wirkung auf die Schwere der Dysmenorrhöe festgestellt, die während der Studie fast völlig verschwand. Diese Auswirkungen auf die Blutung wurden als äußerst relevant für die gute Akzeptanz dieses oralen Niedrigdosen-Empfängnisverhütungsmittels betrachtet.

Die kontinuierliche Anwendung von Valette® (30 µg EE + 2 mg Dienogest) über 189 Tage, gefolgt von einer hormonfreien Zwischenzeit von 7 Tagen wurde bei 30 Frauen in einer gynäkologischen Privatpraxis (Wiegratz et al. (Contraception, 2004;69:37 - 42)) studiert. Daraufhin wurden die Frauen über drei Standardzyklen (21 + 7 Tage) behandelt. Unregelmäßige Blutungen (DBB + Schmierblutungen) sowie hormonverbundene Symptome wurden in Tagebüchern aufgezeichnet. Unregelmäßige Blutungen kamen bei 40 % der behandelten Frauen nur einmal vor, während 17 % mehr als zwei Episoden verzeichneten. Während des zweiten Behandlungszyklus (21. - 42. Tag) verzeichneten 53 % der Frauen unregelmäßige Blutungen (hauptsächlich Schmierblutungen). während im vierten Zyklus (63. - 84. Tag) nur 10 % noch Blutungen verzeichneten. Die meisten der Frauen, die mehr als zwei Blutungsepisoden erlitten, waren anfängliche Verwenderinnen (Anfängerinnen). Die geringste Blutungsrate wurde während des fünften Zyklus beobachtet (3 %), die sich während des 147. - 168. Tags (Zyklus 8) auf 17 % erhöhte. Nur 50 % der behandelten Frauen bekamen während der 7-tägigen hormonfreien Zwischenzeit, gefolgt von dem 189-Tage-Langzeitzyklus Entziehungsblutungen (Menstruation).
Alle Frauen unterzogen sich der 189-Tage-OC-Langzeitbehandlung bis zum Schluss. Jedoch entschlossen sich nur 18 Frauen (60 %), mit dem Langzeitbehandlungsschema fortzufahren, während 4 (13 %) der Frauen jeweils entweder zur normalen Zyklusanwendung zurückkehrten oder zu einem anderen OC übergingen.
In der gleichen Veröffentlichung haben Wiegratz et al. von den Ergebnissen einer Untersuchung unter deutschen Frauen und Gynäkologen bezüglich ihrer Einstellung einer Langzyklusbehandlung mit oralen Empfängnisverhütungsmitteln gegenüber berichtet. Es wurde festgestellt. dass nur 26 - 35 % der befragten Frauen im Alter zwischen 15 und 49 Jahren regelmäßige Menstrualzyklen (einmal monatlich) haben wollten, während 5 - 15 % 4 Menstruationen pro Jahr vorzogen. Nur 11 - 14 % Frauen sind gewillt, die Menstruation kontinuierlich oder für längere Zeitspannen zu unterdrücken, während 32 - 54 % die Menstrualblutung nur sporadisch unterdrücken würden. Die Gründe für die Nichtanwendung von Langzeitzyklus-OCs umfassten die Angst, schwanger zu werden (43 - 72 %), und die Angst vor negativen Auswirkungen (50 - 62 %).

Dem Stand der Technik auf dem Gebiet von Langzeitzyklen-Behandlungsschemen (vergleiche oben) entsprechend wurde angenommen, dass stabile Festlangzeitzyklen (d.h. eine akzeptable Blutungskontrolle) selbst während des ersten Jahres der kontinuierlichen OC-Verabreichung erzielt und aufrechterhalten werden könnten, gleichgültig, welches Progestin verwendet wird.
Jedoch bieten im Gegensatz zu den Ansprüchen von Hodgen (US 5,898,032) OCs mit Standardprogestinen keine verbesserte Kontrolle der Endometriumblutung, im Vergleich mit den jeweiligen Standardzyklusprodukten, bei der Langzeitzyklusanwendung.
So haben verschiedene unabhängige klinische Studien mit Festlangzeitzyklus-OCs eine unakzeptable Blutungskongtrolle bei Standardprogestinen wie beispielsweise Levonorgestrel (Anderson et al., Kovacs et al.) und Desogestrel (Cachrimanidou et al.), wie von Hodgen offenbart, aufgezeigt. Bemerkenswertesterweise wurden sehr hohe Ausscheidungsraten bei diesen Studien vor allem auf Grund von Blutungsproblemen (d.h. hohen Intermenstrualblutungsraten) beobachtet.

Verschiedene Forscher haben das Problem der Intermenstrualblutungen während der Anwendung von Fastlangzeitzyklus-OC untersucht. So offenbart Hesch (US 6,500,814) die kontinuierliche Anwendung von Niedrigdosen-OCs (1 - 20 µg EE/Tag) für eine Zeitspanne von mehr als 110 Tagen, durch die die Intermenstrualblutung verhindert wird. Es werden verschiedene Progestine (Dienogest ist nicht enthalten) beansprucht.

Andererseits offenbart Kulmann (WO 02/22110) ein Langzeitbehandlungsschema, das durch eine Reihenfolge aufeinanderfolgender Festlangzeitzyklen wachsender Dauer gekennzeichnet ist. Dieses Behandlungsschema umgeht anscheinend die Probleme der Festlangzeitzyklus-Behandlungsschemen, gleichgültig, welches Progestin (einschließlich Dienogest) verwendet wird.

Schließlich boten Sulak et al. (siehe oben) Frauen die Wahl, entweder die Anwendung von OC über eine spezifische Anzahl von Wochen (z.B. 6, 9 oder 12) oder bis eine Intermenstrualblutung zu beobachten war, zu verlängern, 3 - 7 Tage auszusetzen und erneut damit zu beginnen. Einphasenpillen mit verschiedenen Progestinen (Norethindron, Levonorgestrel, Norgestimat oder Desogestrel) wurden dabei verwendet. Blutungsprobleme erwiesen sich immer noch als der Hauptgrund für das Abbrechen der Langzeitzyklusanwendung. Es wurde festgestellt, dass der Typ des verwendeten OC keine signifikante Wirkung auf die Fortsetzung der Langzeit-OC-Anwendung ausübt.

Bisher haben keine der zur Verfügung stehenden Langzeitzyklus-OCs (Zusammensetzungen und Methoden) sich als geeignet erwiesen, die lästigen Intermenstrualblutungen (Durchbruchblutung und/oder Schmierblutung), die bei kontinuierlicher Anwendung von OC beobachtet werden, zu eliminieren. Daher ist eine signifikante Reduzierung der Gesamtanzahl von Blutungstagen im Vergleich mit den entsprechenden Standardzyklus-0Cs bei diesen Produkten nicht angezeigt worden.

Detaillierte Offenbarung der Erfindung

Die Aufgabe der vorliegenden Erfindung besteht in der Bereitstellung einer Methode/Regime für die Langzeit-OC-Anwendung mit Vermeidung der Nachteile der konventionellen Methoden/Regime der Langzeit-OC-Anwendung.
Es sollte eine neue Methode/Regime zu einer signifikanten Reduzierung der Gesamtanzahl von Blutungstagen (pro Jahr) im Vergleich mit der entsprechenden Anwendung von Standardzyklus-OC (Überlegenheit bezüglich der Blutung) führen. In einigen Fällen wird die relative Anzahl von Intermenstrualblutungstagen ebenfalls signifikant reduziert. Dieses verbesserte Blutungsbild sollte zu einer bemerkenswerten Reduzierung der Abbrechungs- (Ausscheidungs-) Rate führen.
Des Weiteren sollte eine derartige Methode/Regime einen bestimmten Grad an Flexibilität bezüglich seiner Dauer bieten, um es den Frauen zu erlauben, das Behandlungsschema ihren spezifischen biologisch/medizinischen und individuellen Bedürfnissen anzupassen. Ein derartiges Behandlungsschema würde es erlauben, dem Wunsch der Mehrzahl der Frauen zu entsprechen, die Häufigkeit der Menstrualblutung auf weniger als einmal pro Monat zu reduzieren.
Schließlich sollte eine derartige Methode den Patientinnen zusätzliche Vorteile bieten (z.B. Aknebehandlung), die nicht direkt mit der Blutungshäufigkeit verbunden sind.

Die Aufgabe wird erfindungsgemäße durch ein Verfahren für die weiblichen hormonale Empfängnisverhütung gelöst, wobei eine Einphasenzubereitung von Dienogest in einer täglichen Menge von 0,5 - 3,0 mg und Ethinylestradiol in einer täglichen Menge von 10 - 30 µg oder einem synthetischen oder natürlichen Estrogens, welches in estrogener Wirksamkeit äquivalent zu 10 - 30 µg Ethinylestradiol ist, über eine Zeitspanne von n × 21 Tagen, wobei n = 2 bis 5 ist, gefolgt von einer 7-tägigen Pillenpause, kontinuierlich verabreicht wird.

Vorzugsweise wird dabei eine Pillenpause nach 42, 63 oder 84 Tagen eingeschaltet.

Ferner wird bevorzugt, dass n = 4 (d.h. 84 Tage) ist.

Auch wird bevorzugt, dass die tägliche Menge Dienogest 1,0 bis 2,5 mg beträgt, besonders bevorzugt 2,0 mg Dienogest.

Die tägliche Menge Ethinylestradiol beträgt 20 bis 30 µg oder synthetisches oder natürliches Estrogen , welches in estrogener Wirksamkeit äquivalent zu 10 - 30 µg Ethinylestradiol ist, bevorzugt 30 µg Ethinylestradiol oder eines in synthetischen oder natürlichen Estrogens, in estrogener Wirksamkeit äquivalent zu 30 µg Ethinylestradiol.

Die Einphasenzubereitung wird vorzugsweise in Form von täglichen oralen Dosiereinheiten verabreicht.

Die erfindungsgemäßen verfahren können auch zur Behandlung von Akne, Dysmenorrhöe und /oder Endometriose eingesetzt werden

Auch wird eine pharmazeutische Packung beansprucht, umfassend:
a) eine Einphasenzubereitung von Dienogest in einer täglichen Menge von 0,5 - 3,0 mg und Ethinylestradiol in einer täglichen Menge von 10 - 30 µg oder einem synthetischen oder natürlichen Estrogens, welches in estrogener Wirksamkeit äquivalent zu 10 - 30 µg Ethinylestradiol ist,
b) wobei die Anzahl einzelner Dosiereinheiten in der Zusammensetzung so ausgewählt wird, dass die Methode der hormonalen Empfängnisverhütung nach einem der Ansprüche 1 bis 8 erreicht wird, und
c) Patientinnenanleitungen für die Anwendung der Einphasenzubereitung der Methode für die weibliche hormonale Empfängnisverhütung nach einem der Ansprüche 1 bis 8.

Es konnte gezeigt werden, dass das Problem der Intermenstrualblutung während der festgelegten Anwendung hormonaler Langzeitempfängnisverhütungsmittel bei einer Frau überraschenderweise durch eine neue Empfängnisverhütungsmethode vermieden werden kann, die die kontinuierliche Verabreichung eines Dienogest (DNG) und Ethinylestradiol (EE) enthaltenden Einphasen-Empfängnisverhütungsmittels über eine Zeitspanne von n x 21 Tagen umfasst, wobei n = 2 bis 5 (d.h. 42 - 105 Tage) ist.
Die Erfindung betrifft ein Verfahren für die weibliche hormonale Empfängnisverhütung, welches die kontinuierliche Verabreichung einer Einphasenzubereitung von Dienogest in einer täglichen Menge von 0,5 - 3,0 mg und Ethinylestradiol in einer täglichen Menge von 10 bis 30 µg einer Frau für eine erste Mindestzeitspanne von 42 Tagen umfasst. Nach dieser ersten Mindestzeitspanne kann die Frau eine 7-tägige Pillenpause (hormonfreie Zeitspanne, d.h. kein Einnehmen oder Einnehmen von Placebos) vor allem dann wählen, wenn sie während der vorhergehenden 21 Tage, noch bevorzugter während der letzten 7 Tage unakzeptable Blutungen festgestellt hat. Die Pillenpause des erfindungsgemäßen Verfahrens entsprechend erlaubt es den Frauen, sich schließlich bei Anwendung des Langzeitzyklus-Behandlungsschemas zu stabilisieren, so dass die maximale Langzeitzyklusdauer ohne inakzeptable Intermenstrualblutungen erzielt werden kann. Als Alternative können die Frauen mit dem Langzeitbehandlungsschema für mehrere 21 Tagesperioden fortfahren, bis sie die maximale Zyklusdauer von 105 Tagen (5 x 21 Tagen) erreicht haben. Zu diesem Zeitpunkt ist eine 7-tägige Pillenpause obligatorisch, Zwischen dem 42. und dem 105. Tag können die Frauen, die dem erfindungsgemäßen Langzeitzyklus-Behandlungsschema nach behandelt werden, freiwillig eine 7-Tage-Pillenpause nach 63 (3 x 21) und 84 (4 x 21) Tagen einlegen, je nach ihrer spezifischen medizinisch/biologischen Situation (z.B. Blutungsprobleme, Schwangerschaftsausschluss)/persönlichen Interessen/Bedürfnissen (z.B. Urlaub, Sportereignisse, Prüfungen). In bevorzugten Fällen ist die Langzeitzyklusdauer auf 84 Tage (4 x 21) beschränkt, gefolgt von einer siebentägigen Pillenpause.
Nach einer Pillenpause (hormonfreien Phase) muss die Patientin wieder von vorne anfangen, indem sie die aktive Pille für mindestens die Mindesteinnahmezeit einnimmt, bevor die nächste Pause stattfindet.
Das erfindungsgemäße Verfahren führt zu einer bemerkenswert geringeren, Blutungsproblemen zuzuschreibenden Ausscheidungsrate in klinischen Situationen im Vergleich mit z.B. Seasonale®. Aus diesem Grund führt ein derartiges Verfahren auch zu einer hohen Einhaltung und einer hohen Akzeptanz durch die diese Empfängnisverhütungsmethode anwendende Patientin. Das erfindungsgemäße Verfahren erlaubt es insbesondere OC-Anfängern oder Überwechslern, sich bezüglich des Lanzeitzyklus-OCs so zu stabilisieren, dass sie, falls erwünscht, die Behandlung über die maximale Zeitspanne (Dauer) ausdehnen können.

Das erfindungsgemäße Verfahren wird in der Weise angewendet, in dem ein Produkt auf herkömmliche Weise formuliert und verabreicht wird. D.h. alle Standardanweridungswege, einschließlich der verschiedenen bekannten Typen von Arzneimittelzubereitungen (Abgabesystemen), z.B. transdermalen Pflastern, IUS und Vaginalringen, sowie andere, bei der hormonalen Empfängnisverhütung anwendbare Formulierungen, können zur Ausführung der Erfindung verwendet werden.
Der orale Verabreichungsweg wird vorgezogen.

Die Vorteile des erfindungsgemäßen Verfahrens zur Empfängnisverhütung umfassen:
- die Erzielung einer signifikant verbesserten Blutungsrate (= Reduzierung der Gesamtanzahl von Blutungstagen) im Vergleich mit dem 28-Tage-Standardzyklus- (z.B. 21 + 7) Produkt, selbst während des ersten Jahres der Verabreichung und
- einfaches Behandlungsschema mit Wochenplan (mit jeweils demselben Anfangstag),
- bessere Einhaltung/reduzierte Abbrechungsrate und/oder
- Reduzierung der Nebenwirkungen (z.B. Übelkeit, Kopfschmerzen, Blutungen) und/oder
- Besserung von Hautbeschwerden (Akne) und/oder
- Gleichzeitige Behandlung von Dysmenorrhöe und Endometriose möglich.
- bessere Lebensqualität.

Der flexible Anwendungsplan (2 - 5 x 21 Tage) gestattet es den Frauen, das Behandlungsschema (d.h. die maximale Dauer) ihrer spezifischen medizinischen/biologischen Situation (z.B. Blutungsproblemen, Ausschluss von Schwangerschaft)/persönlichen Interessen/Bedürfnissen (z.B. Urlaub, Sportereignissen. Prüfungen) entsprechend anzupassen. Diese Flexibilität ist bei denjenigen Frauen besonders vorteilhaft, die von anderen OCs überwechseln oder die zum ersten Mal mit einem OC beginnen, da von diesen Patientinnen bekannt ist, dass sie oft häufige Intermenstrualblutungen durchmachen, die zu einem Abbrechen der (Langzeit-) OC-Anwendung führen können.

### Beispiel 1

Das folgende klinische Protokoll wird durchgeführt, um das für die erfindungsgemäße Methode erhaltene Blutungsprofil zu untersuchen. Dieses Protokoll wird vorgeschlagen, um die überlegenen Blutungseigenschaften des EE/DNG-Langzeitzyklus-OC im Vergleich mit dem entsprechenden Standardzyklus-OC (21 + 7 Tage) aufzuzeigen.

Eine ein Jahr dauernde, offene, randomisierte Multizenter-Parallelgruppen-Vergleichsstudie der Phase III an 1.100 gesunden, jungen, fertilen Frauen (400 bewertbare Fälle pro Gruppe) im Alter von 18 - 40 Jahren wird mit einem der erfindungsgemäßen Methode entsprechenden OC durchgeführt. Das OC enthält 2 mg Dienogest (DNG) and 30 µg Ethinylestradiol (EE) (Celimona®/Valette®).

Bei dem vorliegenden Protokoll werden 4 Langzeitzyklen von 84 Tagen, gefolgt von einer 7-tägigen pillenfreien Pause (keine Einnahme) mit 13 herkömmlichen 21-Tagezyklen, gefolgt von jeweils einer 7-tägigen pillenfreien Pause, verglichen. Aus Regelungsgründen müssen alle der dem Langzeitzyklus unterworfenen Frauen sich an den 84-Tage-Plan halten, um eine ausreichende Sicherheitsexposition/Daten für die maximal beabsichtigte Anwendungsdauer zu erhalten.

Es werden Standardeinschluss- und -ausschlusskriterien für OC-Studien angewendet.

Der primäre Studienparameter ist die Anzahl von Tagen mit intrazyklischer Blutung während eines Jahres.

Außerdem werden die Blutungsbilder und Zykluskontrollparameter durch ein auf Papier aufgezeichnetes Tagebuch beurteilt. Das Mindestziel besteht darin, eine signifikante Reduzierung der Gesamtanzahl von Blutungstagen während eines Jahres (d.h. 364 Tagen) für die Langzeitzyklus- (84 + 7 Tage) im Vergleich mit der Standardzyklus- (21 + 7 Tage) Anwendung aufzuzeigen.

Die Anzahl unbeabsichtigter Schwangerschaften wird abgeschätzt (Pearl-Index, Lebenstabellenanalyse).

Außerdem werden Standardsicherheitsparameter für OC untersucht.

## Patentansprüche

1. Verfahren für die weiblichen hormonale Empfängnisverhütung, wobei eine Einphasenzubereitung von Dienogest in einer täglichen Menge von 0,5 - 3,0 mg und Ethinylestradiol in einer täglichen Menge von 10 - 30 µg oder einem synthetischen oder natürlichen Estrogens, welches in estrogener Wirksamkeit äquivalent zu 10 - 30 µg Ethinylestradiol ist, über eine Zeitspanne von n x 21 Tagen, wobei n = 2 bis 5 ist, gefolgt von einer 7-tägigen Pillenpause,kontinuierlich verabreicht wird.

2. Verfahren nach Anspruch 1, wobei eine Pillenpause nach 42, 63 oder 84 Tagen eingeschaltet wird.

3. Verfahren nach Anspruch 1, wobei n = 4 (d.h. 84 Tage) ist.

4. Verfahren nach einem der Ansprüche 1 bis 3, wobei die tägliche Menge Dienogest 1,0 bis 2,5 mg beträgt.

5. Verfahren nach Anspruch 4, wobei die tägliche Menge Dienogest 2,0 mg beträgt.

6. Verfahren nach einem der Ansprüche 1 bis 5, wobei die tägliche Menge Ethinylestradiol 20 bis 30 µg oder synthetisches oder natürliches Estrogen beträgt, welches in estrogener Wirksamkeit äquivalent zu 10 - 30 µg Ethinylestradiol ist.

7. Verfahren nach Anspruch 6, wobei die tägliche Menge Ethinylestradiol oder eines in seiner wirksamkeit äquivalenten Estrogens 30 µg beträgt.

8. Verfahren nach den Ansprüchen 1 bis 7, wobei die Einphasenzubereitung in Form von täglichen oralen Dosiereinheiten verabreicht wird.

9. Verfahren nach einem der Ansprüche 1 bis 8 zur Behandlung von Akne

10. Verfahren nach einem der Ansprüche 1bis 8 zur Behandlung von Dysmenorrhöe

11. Verfahren nach einem der Ansprüche 1bis 8 zur Behandlung von Endometriose

12. Pharmazeutische Packung, angepasst für die orale Verabreichung, umfassend:
a) eine Einphasenzubereitung von Dienogest in einer täglichen Menge von 0,5 - 3,0 mg und Ethinylestradiol in einer täglichen Menge von 10 - 30 µg oder einem synthetischen oder natürlichen Estrogens, welches in estrogener Wirksamkeit äquivalent zu 10 - 30 µg Ethinylestradiol ist,
b) wobei die Anzahl einzelner Dosiereinheiten in der Zusammensetzung so ausgewählt wird, dass die Methode der hormonalen Empfängnisverhütung nach einem der Ansprüche 1 bis 8 erreicht wird, und
c) Patientinnenanleitungen für die Anwendung der Einphasenzubereitung der Methode für die weibliche hormonale Empfängnisverhütung nach einem der Ansprüche 1 bis 8.
